# EUROPEAN PATENT APPLICATION

(11) **EP 2 202 519 A1**
(43) Date of publication of application: **30.06.2010**
(21) Application number: 08172992.3
(22) Date of filing: 29.12.2008
(51) Int. Cl.: G01N 33/543

(54) **A detector system for identifying substances in a sample**

(71) Applicant: Danmarks Tekniske Universitet - DTU, 2800 Kongens Lyngby (DK)
(72) Inventor: Jakobsen, Michael Linde, 4000, Roskilde (DK); Pedersen, Henrik Chresten, 4040, Jyllinge (DK); Hanson, Vagn Steen Grüner, 4640, Fakse (DK); Dam-Hansen, Carsten, 2630, Taastrup (DK)
(74) Representative: Kitchen, Steven Richard

(57) **Abstract**

A detector system for identifying substances in a sample is described. The detector system comprises: a plurality of cantilevers (330) arranged in a cantilever array, the cantilevers being arranged so as to be able to bend along a cantilever axis, wherein at least a number of the cantilevers are provided with a recognition layer for identifying a substance in the sample, each of the number of cantilevers being adapted to bend along its cantilever axis if the recognition layer reacts to a substance corresponding to the recognition layer, and an optical system for probing a deflection of the cantilevers. The optical system includes a common-path interferometer setup comprising a transmitter part and a receiver part. The transmitter part comprises light source means (310) for emitting spatially coherent light and arranged so as to illuminate at least parts of the cantilevers of the cantilever array. The receiver part comprises a detector array (334,336) and a receiver diffractive optical element (318) adapted so as to collect light reflected from the cantilever array and for each cantilever to form at least two spatially overlapping images of said parts of the cantilever so that interference between the two spatially overlapping images is formed at and measured by at least one separate detector (334) of the detector array.

## Description

The invention relates to a detector system for identifying substances in a sample, wherein the detector system comprises: a plurality of cantilevers arranged in an array, the cantilevers being arranged so as to be able to bend along a cantilever axis, wherein at least a number of the cantilevers is provided with a recognition layer for identifying a substance in the sample, each of the number of cantilevers being adapted to bend along its cantilever axis if the recognition layer reacts to a substance corresponding to the recognition layer, and an optical system for probing a deflection of the cantilevers.

The use of micro-fabricated cantilevers for fast detection of substances in a sample is well-known in the art. Sensors or detector systems based on such cantilevers can for instance be used for surface analysis, chemical sensing, gas sensing and for detection of microorganisms, such as bacteria, fungi, cells or other similar organisms.

The micro-fabricated cantilevers are usually fixed at one end to a solid support and are able to bend freely at the other end. The bending or oscillation of the cantilever can then be used to determine the quantity of a given substance in a sample passing through a chamber containing the cantilevers. The cantilevers are provided with recognition layers, such as chemical coatings, e.g. by use of polymers, which are able to bind or adsorb molecules more or less selectively. The binding or adsorption entails a change in surface stress and/or change in mass, thus causing the cantilever to bend or oscillate at a different resonance frequency. The degree of bending or the frequency and/or amplitude of oscillation can be used to determine the quantity of the given substance in the sample. By using an array of cantilevers having different recognition layers, it is possible to detect a plurality of different substances simultaneously.

Furthermore, it is known in the art to use optical systems for probing the degree of deflection or the frequency of oscillation of the cantilever. Such optical systems are often similar to detection systems known from atomic force microscope (AFM) setups, in which a laser beam is sent to the surface of the cantilever at an angle, and the reflected beam is measured by a detector array. The bending of the cantilever is detected by the change in the reflection angle of the laser beam. However, "cantilever readers" based on the AFM principle are quite difficult to align, in particular, when cantilevers in an array are detected individually.

It is an object of the present invention to obtain a new detector system, which overcomes or ameliorates at least one of the disadvantages of the prior art or which provides a useful alternative.

According to a first aspect, this is obtained by the optical system including a common-path interferometer setup comprising a transmitter part and a receiver part, the transmitter part comprising light source means for emitting spatial coherent light and arranged so as to illuminate at least parts of the cantilevers of the cantilever array, and the receiver part comprising a detector array and a receiver diffractive optical element adapted so as to collect light reflected from the cantilever array and for each cantilever forming at least two spatially overlapping images of said parts of the cantilever so that interference between the two spatially overlapping images is formed at - and measured by - at least one separate detector of the detector array.

Thus, it is recognised that the detector system provides an array of probing beams focused onto an array of cantilevers having different recognition layers. Thus, a sample can expose the cantilever array, whereby the sample quickly and efficiently may be analysed for agents or substances present in the sample. Furthermore, the detector system employs a common-path interferometry scheme, in which optical functions, such as beam splitting, and focusing and/or re-collimation of the probing beams, may be integrated in the same diffractive optical element. Since the probing beams are following substantially parallel paths (or common paths), the system is substantially insensitive to mechanical vibrations, since the vibrations are substantially identical for all paths. Thereby, only out-of-plane deflections or bending is measured corresponding to the differential displacement between different illuminated areas of the cantilevers. Furthermore, the setup is as such self-aligning, since all the crucial parts of the optical system are integrated into the same holographic or diffractive optical element. Besides, the use of diffractive optical elements in both the transmitter and receiver makes the system self-adjusting with respect to a change in wavelength of the light source means. In addition, the calibration does not call for absolute knowledge of the laser wavelength.

Furthermore, by integrating the optical functions of the receiver part in a receiver diffractive optical element, it is possible to obtain quadrature signals - i.e. signals being displaced 90 degrees in phase in relation to the main interference signal - in sidebands due to the different diffraction orders from the diffractive optical element. Thus, it is ensured that the sensitivity of the optical system is always high, since it is ensured that at least one of the detectors in the detector array always measures a signal with high dynamics. Furthermore, it is possible to determine the direction of deflection of the cantilevers.

The diffractive optical element can be implemented as a hologram and may be used in a transmissive or reflective configuration. Often, a master of the hologram is implemented by utilising a writing setup for exposing photoresist and subsequent development. The master may be replicated by use of a shim or a stamper and copying the structure of the master to a workable material, such as polymers, e.g. polycarbonate. The writing setup for fabricating the master and the replication procedure are well-known to a person skilled in the art.

By light source means is meant any light source having a coherence length useful for common-path interferometry. The wavelength of the laser beam may lie in the ultraviolet range, the visible range, or the infrared range.

By common-path interferometer is meant an optical system, wherein the beams in the transmitter part follow substantially the same path, for instance parallel paths, and wherein the beams in the receiver part follow substantially the same path (as opposed to for instance a Michelson interferometer, which has a reference arm).

By bending is meant that the surface of the cantilever is deflected away from the cantilever axis such that the optical system probes out-of-plane deflections of the cantilevers of the cantilever array.

By images or imaging is not meant that the cantilevers and/or the detector array necessarily have to be arranged exactly in the image plane of an imaging system. In the context of the present invention, this only means that the light reflected from the cantilevers is brought to interfere by use of the diffractive optical element(s), and that the detectors of the detector array are arranged in a position, where the interference of the reflected light can be measured.

According to an advantageous embodiment, the light source means are arranged so as to illuminate at least a substantial part of the cantilevers, wherein the receiver diffractive element is adapted so as to, for each cantilever, form displaced images of said substantial part of the cantilever at the detector array. Thereby, a particular simple setup is provided, wherein the cantilevers may be illuminated directly by the light source means, and where images of said substantial parts of the cantilevers are brought to partially overlap, thus creating a pattern of interference fringes. By carefully arranging the detector arrays measuring the interference fringes, it is possible to obtain quadrature signals and thus ensure high sensitivity of the system.

According to another advantageous embodiment, the transmitter part further comprises a transmitter diffractive optical element arranged so as to split the light beam up into a plurality of probing beam sets and directing them to the cantilevers of the cantilever array, each of the individual probing beam sets comprising a number of substantially parallel probing beams including at least a first probing beam focused onto a first area of a corresponding cantilever, and a second probing beam focused onto a second area of the corresponding cantilever, the first area and the second area being positioned at a distance from each other along the cantilever axis, and wherein the receiver part comprises a detector array and a receiver diffractive optical element adapted so as to collect light from the probing beam sets reflected from the cantilever array and directing the collected light towards the detector array so that, for each probing beam set, interference between the first reflected probing beam and the second reflected probing beam is measured by at least one separate detector.

Thereby, a particularly advantageous embodiment is provided, wherein the transmitter part of the optical system is adapted to form an array of probing beams focused onto small areas of the cantilevers of the cantilever array, and wherein the receiver part of the optical system forms overlapping images of the probing beam spots, such that probe beams of each of the probing beam sets are reflected and brought to interfere at the detector array. Thus, the out-of-plane displacement between the first area and the second area of each cantilever can be measured with high precision. Thereby, it is also possible to obtain a high precision quantity estimation of the particular substances to be detected.

According to another advantageous embodiment, at least one of the cantilevers is a reference cantilever. The reference cantilever is to be provided without a recognition layer, or, it may be passivated to make it non-reactive to certain chemical or biological substances. Thereby, the system may be automatically adjusted to for instance thermal changes of the detector system or fluctuations in the wavelength or direction of propagation of the light source means. In this manner, the precision of the system may be improved even further.

The optical system may of course also be used for other applications, wherein the out-of-plane displacement of an object is to be probed at a plurality of areas. Thus, according to a second aspect, the invention provides: an optical system including a common-path interferometer setup comprising a transmitter part and a receiver part, wherein the transmitter part comprises light source means for emitting spatial coherent light beam and a transmitter diffractive optical element arranged so as to direct and split the laser beam up into a plurality of probing beam sets, each of the individual probing beam sets comprising a number of substantially parallel probing beams including at least a first probing beam for being focused onto a first area of an object, and a second probing beam for being focused onto a second area of the object, the first area and the second area being positioned at a distance from each other, and wherein the receiver part comprises a detector array and a receiver diffractive optical element adapted so as to collect light from the probing beam sets reflected from the object and directing the collected light towards the detector array so that, for each probing beam set, interference between the first reflected probing beam and the second reflected probing beam is measured by at least one separate detector.

In the following, a number of advantageous embodiments are described. The embodiments relate to both the first and the second aspect of the invention, i.e. to the detector system and the optical system itself.

According to a particularly advantageous embodiment, the light source means comprises a laser, such as a laser diode or a vertical cavity surface emitting laser (VCSEL), and a collimating lens. Such lasers provide inexpensive laser means for providing a collimated laser beam. Collimated laser beams are preferred, since the optical components of the optical system can be kept at a restricted size. Furthermore, it makes the implementation and alignment of the diffractive optical element simpler.

However, since the setup is substantially wavelength-independent and the setup utilises a common-path interferometry scheme, the light source means only need to have a limited spatial coherence. Thus, a narrowband light emitting diode (LEDs) may be sufficient, e.g. an organic light emitting diode (OLED) or a NCSEL.

According to yet another particularly advantageous embodiment, the light source means comprises a laser array, preferably a VCSEL array or a laser diode array. By using laser arrays, a particularly simple method of implementing the array of probing beams is provided. The laser diode array provides a plurality of laser beams, and each laser beam may be diverted and split up into probing beams by one or more diffractive optical elements.

In one embodiment, the light source means further comprises a collimating lens arranged so as to provide a number of substantially collimated light beams. By using a one-dimensional laser array (diode array or VCSEL array) and a single collimating lens, it is possible to obtain a number of spatially overlapping, but angularly separated laser beams emitted from the light source means. These overlapping, collimated beams are then sent to the transmitter diffractive optical element. Due to the implementation of the transmitter diffractive optical element, each of the incoming beams is split up and focused onto at least two separate spots on its corresponding cantilever. Thus, the collimating lens and the transmitter diffractive optical element form an imaging system, wherein the one-dimensional laser array is double-imaged onto essentially two arrays. Hence, in this manner, laser element 1 is double-imaged onto cantilever 1, giving rise to two separated spots on cantilever 1. Similarly, laser element 2 is double-imaged onto cantilever 2, giving rise to two separated spots on cantilever 2, etc. Hence, cross talk is largely avoided. Thus, according to an advantageous embodiment, a plurality of beams are split up into probing beam sets by a single transmitter diffractive optical element, e.g. the beams from a single laser array. It is of course possible to use several laser arrays and a separate transmitter diffractive optical element for each of the laser arrays.

Furthermore, according to the embodiment just described, a lens positioned in front of the detector array in the receiver system may form an imaging system together with the receiver diffractive optical element, in such a way that the light reflected from the two arrays of spots on the cantilevers is double-imaged onto the detector array. In this manner, overlapping and interfering spots will be formed at the detectors, in such a way that essentially all reflected light from cantilever 1 will be collected and double-imaged onto detector elements 1, essentially all reflected light from cantilever 2 will be collected and double-imaged onto detector elements 2, etc. Hence, cross-talk is largely avoided.

According to an alternative embodiment, the light source means comprises a lens array comprising collimating lenses. Thereby, a particular simple method of obtaining a plurality of collimated, angularly separated, and possibly non-overlapping laser beams is provided.

In another embodiment, the light source means comprises beam splitter means for splitting the laser beam up into a plurality of laser beams, the plurality of laser beams preferably being parallel. Thus, an alternative to utilising a laser diode array or a VCSEL array is provided. It is recognised that the beam splitting may be achieved in a number of ways, for instance by the beam splitter means comprising a Wollaston prism and a condensing lens. The beam splitting means can also be implemented by use of a first grating for splitting the beam up into a number of individual beams, and optionally a second grating for aligning the individual laser beams in parallel.

According to a first advantageous embodiment, the transmitter diffractive optical element or elements are arranged in a first plane, and the receiver diffractive optical element or elements are arranged in a second plane. According to a second advantageous embodiment, the transmitter diffractive optical element or elements and receiver diffractive element or elements are arranged in a common plane. Thereby, a particularly robust system is provided, since alignment of the transmitter diffractive optical element automatically means that the receiver diffractive optical element is aligned.

According to yet another advantageous embodiment, the first plane and/or the second plane are arranged substantially parallel to a cantilever plane, in which the cantilevers are positioned. This provides a particularly simple method of mutually aligning the cantilever plane and the plane comprising the diffractive optical elements. Furthermore, the fabrication of the diffractive optical elements is made simpler, since the focal lengths of the first probing beam and the second probing beam are identical.

According to a first advantageous embodiment, the transmitter diffractive optical element is adapted to emit the probing beams substantially normally to the first plane in response to an incoming collimated beam incident on the first plane in an a first angle to the normal of the first plane. Advantageously, the first angle lies in an interval between 30 and 60 degrees, for instance approximately 45 degrees. Thus, the incoming beam is diverted 45 degrees. It is possible to obtain a higher efficiency for the diffractive optical element by making the diversion angle even higher. Thus, it may be advantageous to emit the probing beams in a second angle to the normal of the first plane, the second angle being e.g. 45 degrees, making the total diversion angle 90 degrees.

According to a particularly advantageous embodiment, the transmitter diffractive optical element and/or the receiver diffractive optical element comprise a first lens element for forming the first probe beam and a second lens element for forming the second probe beam, the first lens element and the second lens element being partially overlapping. Thereby, a particularly simple method is provided, where it is ensured that the reflected light from the first probing beam and the second probing beam is made to interfere at the detector array. Preferably, the lens elements are arranged so that they divert and focus an incoming collimated beam into a focused probing beam. The distance between the lens element and the cantilever plane should correspond to the focal length of the particular lens element. When providing both the transmitter diffractive optical element and the receiver diffractive optical element as partially overlapping lens elements arranged with a distance to the cantilever array corresponding to the focal lengths of the lens elements, it is ensured that the received light is re-collimated and sent towards the detector array as co-propagating, overlapping beams.

The overlapping area of the diffractive optical element comprises two different gratings, and a spatial beating between two different grating constants may be obtained. This can be achieved by double exposing a hologram in a holographic setup. Such a method is for instance described in J. N. Latta and R. V. Pole, "Design techniques for forming 488-nm holographic lenses with reconstruction at 633 nm," Applied Optics 18, 2418-2421 (1979) and other articles by J. N. Latta.

According to another advantageous embodiment, a single diffractive optical element is utilised as both the transmitter diffractive optical element and the receiver diffractive optical element.

According to one embodiment, the transmitter part comprises a transmitter path for incoming light and the receiver part comprises a receiver path for light reflected from the cantilevers, the transmitter path and the receiver path having an overlapping part, wherein the transmitter path and the receiver path are substantially overlapping. A beam splitter may be arranged in both the transmitter path between the laser means and the transmitter diffractive optical element and in the receiver path between the receiver diffractive optical element and the detector array. The beam splitter may for instance be a cube beam splitter or a slab. Thus, it can be ensured that the reflected probing beams are sent to the detector array, at the same time ensuring that transmitted light from the transmission path is not sent to the detector array.

According to a particular simple embodiment, the detector array comprises a CMOS array or a CCD array. Such arrays provide cheap and effective detection of the interference signals, however such sensors typically have a slow response time, which makes them suitable for the detection of only static deflections of the cantilevers.

However, it is not necessary to employ such detector arrays. It is for instance possible to position individual photo detectors in such a way that the interference from one probing beam set is detected by at least one separate photo detector. Such a setup is particularly advantageous where the quantity of a substance in the sample is to be measured based on the vibration frequency of the cantilever. Such vibrations typically lie in the kHz range. The cantilevers may for instance be brought to oscillation by use of a piezo-electric transducer.

According to an advantageous embodiment, at least two detectors are utilised for each cantilever in order to measure two separate interference signals. Preferably, the two detectors are arranged so as to measure two interference signals being mutually shifted 90 degrees in phase.

According to another embodiment, a number of the probing beam sets comprises a third probing beam focused onto a third area of the corresponding cantilever, the third area being positioned at a first distance from the first area along the cantilever axis and at a second distance from the second area along the cantilever axis. If only two probing beams for each cantilever are used, it is only possible to obtain an unambiguous, absolute differential displacement measurement of the cantilever between the first area and the second area corresponding to half a wavelength of the laser means used due to the common-path interferometry scheme employed. By utilising a third probing beam and properly choosing the distance between adjacent probing beams, it is possible to obtain two separate interference measurements (between light reflected from adjacent beams), where the two interference measurements have different spatial frequency dependencies. By measuring the phase difference between the two interference measurements, it is possible to expand the measurement range, in which an absolute differential displacement measurement can be obtained.

According to yet another embodiment, the cantilevers and/or the optical system are arranged on a rotational stage, so that a cantilever plane comprising the cantilevers can be rotationally adjusted in relation to the transmitter diffractive optical element and the receiver diffractive optical element. Thereby, a particular simple method of aligning the cantilever array in relation to the optical system is provided. Furthermore, the cantilevers and/or the optical system may be arranged on a transitional stage so that a distance between a cantilever plane comprising the cantilevers and the diffractive optical elements can be adjusted.

Preferably, the cantilevers are arranged in a housing having a chamber arranged so that the sample to be analysed can pass by the cantilevers, e.g. by having an inlet and an outlet. Furthermore, the chamber may be able to communicate with a second chamber for collecting the sample so that the sample can be disposed of together with the housing containing the cantilevers.

According to an advantageous embodiment, the cantilevers are adapted to bend with a total out-of-plane displacement being less than the wavelength of the light source means used. Advantageously, the total displacement is less than one fourth of the wavelength. The total out-of-plane deflection between the two areas of the cantilever may for instance be in the range between 50 nm and 1000 nm, or between 70 nm and 700 nm, or between 100 nm and 500 nm, e.g. about 200 nm.

The coherence length of the light source means should be at least twice the length of the total out-of-plane deflection. Thus, in a system utilising cantilevers having a maximum total out-of-plane deflection of about 200 nm, the coherence length should be at least 400 nm.

The wavelengths of the different beams may in principle differ. The individual lasers of a laser array can for instance have different wavelengths.

According to yet another advantageous embodiment, the cantilevers are provided with a highly reflective layer. Thereby, it is ensured that highly dynamic interference signals are obtained at the detector plane. One possible way of obtaining a highly reflective layer is by coating the cantilevers with gold. Gold also has the advantage of providing for good linkers for the recognition layers.

The invention is explained in detail below with reference to an embodiment shown in the drawings, in which
Figs. 1 and 2 show schematic views of a first embodiment of a detector system according to the invention, seen from the side,
Fig. 3 shows a schematic view of an embodiment of a light source means for use in the detector system according to the invention, the light source means comprising a VCSEL array and a collimating lens,
Fig. 4a and 4b show schematic views of cantilever arrays for use in the detector system according to the invention,
Fig. 5 shows a schematic view of probe beam sets imaged onto a detector array,
Fig. 6 shows a schematic view of an embodiment of a cantilever setup housing for use in the detector system according to the invention,
Fig. 7 shows a schematic view of a beam splitter means for forming a number of parallel beams,
Fig. 8 and 9 show schematic views of a second embodiment of a detector system according to the invention, seen from the side,
Fig. 10 and 11 show schematic views of a third embodiment of a detector system according to the invention, seen from the side,
Fig. 12 shows a schematic view of a fourth embodiment of a detector system according to the invention, seen from the side,
Fig. 13 shows a schematic view of a diffractive optical element plane used in the fourth embodiment, seen in perspective,
Fig. 14 shows a schematic view of a fifth embodiment of a detector system according to the invention, seen from the side,
Fig. 15 shows a schematic view of a diffractive optical element plane used in the fifth embodiment, seen in perspective,
Fig. 16 shows the bending of a cantilever,
Fig. 17a and 17b show quadrature signals,
Fig. 18 shows typical signal responses related to different cantilevers, and
Fig. 19 shows signals in a spatial heterodyne common-path interferometer.

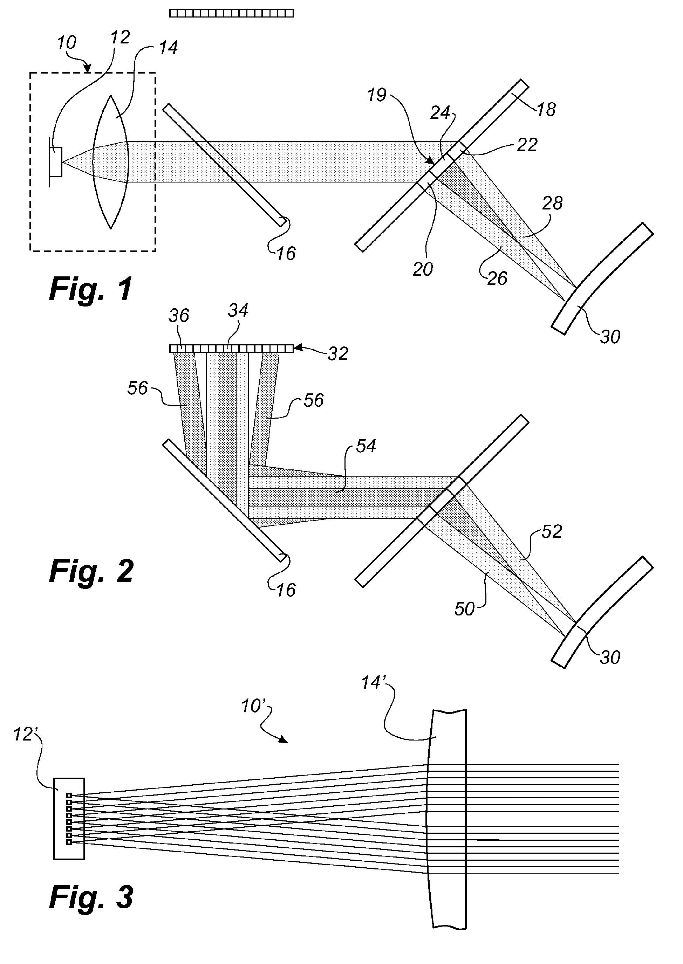

Figs. 1 and 2 show schematic views of a first embodiment of a detector system according to the invention, seen from the side. The detector system comprises an optical system utilising a common-path interferometer setup and a cantilever array comprising a number of cantilevers 30. The optical system comprises a light source means 10, which in turn comprises a VCSEL array 12 and a collimating lens 14. The optical system additionally comprises a beam splitter 16 and a diffractive optical element layer 18 comprising a diffractive optical element 19 being formed as two partially overlapping lens elements, i.e. a first lens element 20 and a second lens element 22 having an overlapping region 24. Furthermore, the optical system comprises a detector array 32, e.g. a CMOS or a CCD array 32.

Fig. 1 shows the optical system when the diffractive optical element 19 functions as a transmitter in the system. Light is emitted from the VCSELs of the VCSEL array 12 and is collimated by a collimating lens 14. The collimated beams are sent to the diffractive optical element 19 at an average angle of for instance 45 degrees. Since the diffractive optical element 19 is composed of two overlapping lens elements 20, 22, the diffractive optical element 19 splits the incoming collimated beams into a number of first probing beams 26 and a number of second probing beams 28, which are focused onto the cantilevers 30. Thus, for each VCSEL of the VCSEL array 12, the collimated beam is split up into a first probing beam 26 and a second probing beam 28, which are focused onto separate areas of the cantilever 30.

Fig. 2 shows the same optical system, when the diffractive optical element 19 functions as a receiver in the common-path interferometer. For each cantilever 30, a first reflected probing beam 50 and a second reflected probing beam 52 are reflected from the cantilever. The reflected beams are collected by the diffractive optical element 19 and are re-collimated and are directed to the detector array 32 via the beam splitter 16 as two co-propagating, partially overlapping beams 54. The interference of the partially overlapping beams 54 can be measured by at least a first detector 34 of the detector array 32. Due to the diffractive optical element being composed of two partially overlapping lens elements, the diffractive optical element 19 provides sidebands 56 also comprising two partially overlapping beams. Thus, the sidebands 56 also provide interference signals, which can be measured by detectors in the detector array 32. The interference signals of the sidebands are shifted approximately -90 and 90 degrees in phase, respectively, compared to the main interference signal measured by the first detector 54. Due to the common-path interferometry setup, the phase difference between the interference signals is substantially independent of the spatial positions of the detectors of the detector array 32.

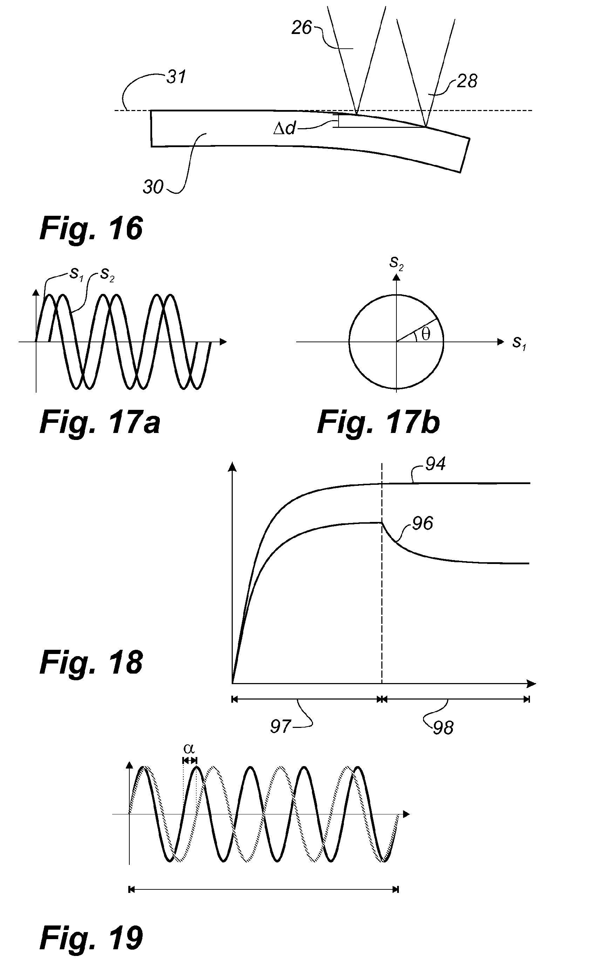

The cantilevers 30 are each provided with recognition layers and will bend due to surface tension if a substance corresponding to the particular recognition layer is present in a sample flowing past the cantilever. Fig. 16 shows an example of the bending of a cantilever 30, where the cantilever bends along a cantilever axis 31. The bending of the cantilever 30 can be measured by use of the probing beams 26, 28. Due to the common-path interferometry setup, common displacement of the first area and the second area of the cantilever 30 is not detected. Instead the bending is measured as an out-of-plane displacement corresponding to the differential displacement Δ*d* from the cantilever axis 31 between the first area and the second area of the cantilever 30.

In the particular setup shown in Fig. 2, only one of the interference signals of the sidebands 56 is used, this signal being measured by a second detector 36 of the detector array. By utilising the main interference signal s₁ measured by the first detector 34 and the interference signal s₂ measured by the second detector 36 and being shifted approximately 90 degrees compared to the main interference signal (also called quadrature signals), a high sensitivity for the common-path interferometer is obtained, since it is ensured that at least one of the interference signals s₁, s₂ is located between inflection tangents as shown in Fig 17a. In practice, the differential deflection of the cantilever can be meaured by calculating the phasor θ = arctan(s₂/s₁) of the interference signals by using both the measured signals s₁, s₂ as shown in Fig. 17b, where the first detector signal and the second detector signal are depicted in a polar plot.

In an advantageous embodiment, a condensing lens is arranged between the beam splitter 16 and the detector array 32 so that the reflected beams are focused on to the detectors of the detector array 32. Furthermore, the cantilever array and/or the optical system can be arranged on a translational stage and/or a rotational stage so as to ensure that the probing beams are all focused onto the cantilevers of the cantilever array.

Fig. 3 shows an example of a light source means 10' for use in the optical system. The light source means 10' comprises a VCSEL array 12' emitting a number of diverging beams. The beams are collimated by a collimating lens 14', which collimates the beams. The VCSELs emit light with a divergence angle of approximately +/- 5 degrees, and may to an observer be perceived as a single beam. Therefore a single diffractive optical element is sufficient for the entire VCSEL array 12'.

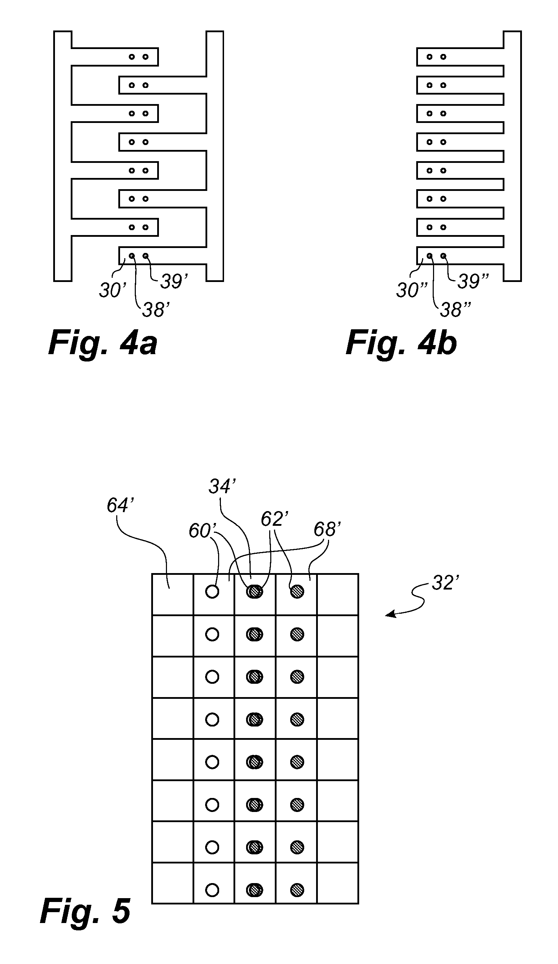

Fig 4a and 4b show examples of cantilever arrays. Fig 4b shows one embodiment, where the cantilevers 30" are mounted to a single solid support at a proximal end and are arranged to bend freely at a distal end. Fig. 4a shows a similar embodiment, where every second cantilever 30' is mounted to a separate solid support. The light from each VCSEL of the VCSEL array is split up by the diffractive optical element and focused onto a first probing beam spot 38', 38" or a first area of the cantilever, and a second probing beam spot 39', 39" or a second area of the cantilever. Thus, it is seen that each element of the VCSEL array is imaged onto two probing beam spots at the corresponding cantilever.

One of the cantilevers is a reference cantilever. The reference cantilever is provided without a recognition layer or is passivated. Thereby, the system may be automatically adjusted to for instance thermal changes of the detector system. In this manner, the precision of the system may be improved even further.

The two arrays of probing beam spots are in turn imaged onto the detector array. Since the diffractive optical element is used as both transmitter and receiver in the system, the two arrays are imaged as two separate, partially overlapping images at the detector array 32'. Thus, the two arrays of probing beams are double imaged onto the detector array 32' so that first reflected probing beams are brought to interfere with second reflected probing beams. This is depicted in Fig. 5, in which it is seen that a first image 60' and a second image 62' of probing beam spots are imaged onto the detector array 32'. It is seen that the detector array 32' comprises a number of detectors 64' measuring no signals, a number of first detectors 34' measuring interference between first reflected and second reflected probing beams, and a number of detectors 68' measuring signals from one probing beam only (and thereby no interference signal). Of course, it is also possible to detect the quadrature signals as previously explained. In this case, the double images have side bands that lead to interference signals at detectors 68' as well.

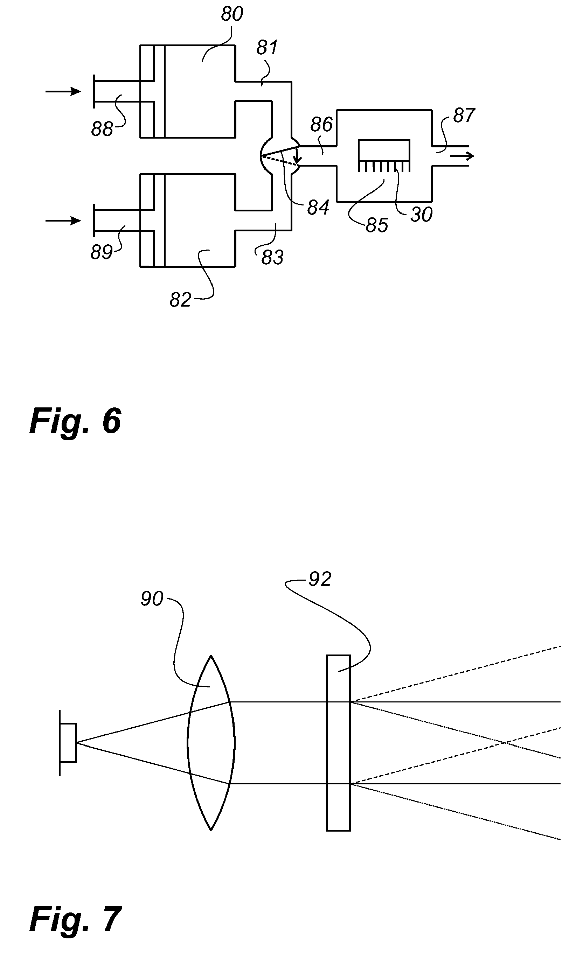

Fig. 6 shows an example of the cantilever setup housing for use in the detector system, in which the cantilever is installed in a flow system that allows alternating exposures of the cantilever sensor to a buffer fluid and a sample fluid. The cantilever setup housing comprises a detection chamber 85, in which the cantilever array with a number of cantilevers 30 is arranged. A sample to be analysed is loaded into a sample chamber 80. The sample chamber 80 may be formed in any conventional way, for instance as a syringe that is activated by applying a force to a piston 88. The sample chamber is connected to the detection chamber 85 via a sample chamber outlet 81, a valve 84, and an inlet 86 to the detection chamber 85. Furthermore, the cantilever setup comprises a buffer chamber 82 containing a buffer. The buffer chamber 82 may be formed in any conventional way, for instance as a syringe that is activated by applying a force to a piston 89. The buffer chamber 82 is connected to the detection chamber 85 via a buffer chamber outlet 83, the valve 84 and the inlet 86 to the detection chamber 85. Once the sample and/or the buffer has passed through the detection chamber 85 it passes through a detection chamber outlet 87 and continues on to a sample collection chamber (not shown). Thereby, the cantilever setup housing together with the sample (and possible buffer) can be disposed of after the sample has been analysed for known substances.

However, it is sufficient to form the cantilever setup housing with the sample chamber only, i.e. without a buffer fluid. Thus, the sample may be inserted in the sample chamber and be drawn into the detection chamber, e.g. by use of capillary forces.

Fig. 17 shows a typical signal response related to two cantilevers. In a first time zone 97, the cantilevers are exposed to the buffer, and in a second time zone 98, the cantilevers are exposed to the sample. It is seen that one cantilever's signal 94 does not change due to exposure from the sample (negative) and another cantilever's signal 96 does change due to exposure from the sample (positive).

Fig. 7 shows an alternative means for providing a number of angularly separated beams from the light source means. In this embodiment, a lens 90 collimates the light from a single light source, after which a grating 92 splits the light into a number of angularly separated beams (only three are shown) by using the zero'th, first, second, and third order diffractions and so forth. The diffractive optical element will then focus and direct each of the angularly separated beams as a double-spot at each cantilever in the array. Thereby a single laser diode, VCSEL, LED or similar can be used instead of an array.

In principle, a similar embodiment can be obtained by providing a number of parallel beams from the light source means. This can for instance be obtained by using a first grating and a second grating arranged in parallel. Thereby a single incoming beam can be split up into a number of parallel beams by using the zero'th, first, second, and third order diffractions and so forth. However, if only a single transmitter diffractive optical element is used, all parallel beams are focused onto the same two spots. Therefore, systems employing parallel beams incident on the diffractive optical element layer need a separate transmitter diffractive optical element for each parallel beam, thus adding unnecessary complexity to the system.

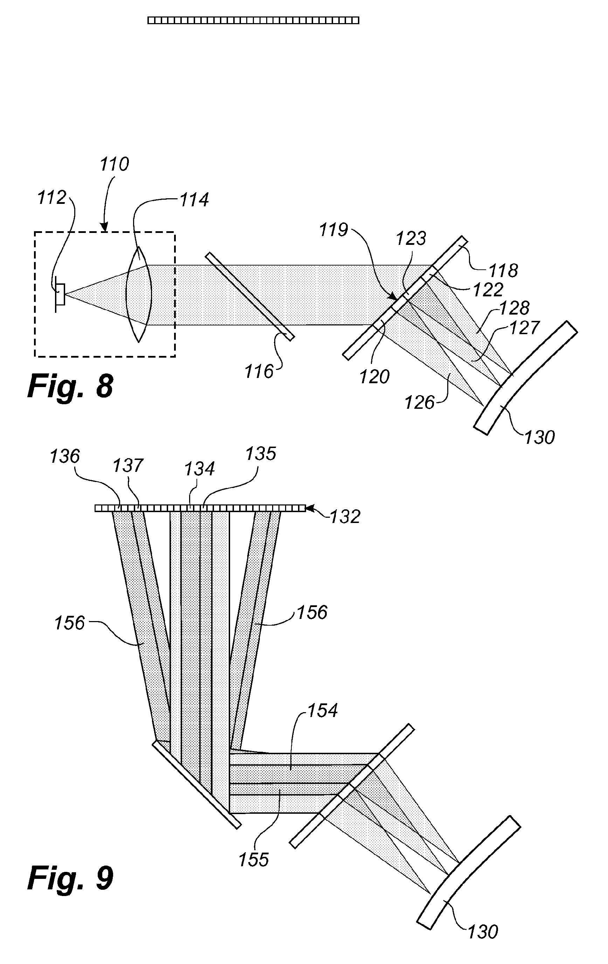

Fig. 8 and Fig. 9 show a second embodiment of a detector system according to the invention, wherein like numerals refer to like parts of the first embodiment shown in Figs. 1 and 2. Therefore, only the difference between the two embodiments is described. The diffractive optical element 119 is composed of three partially overlapping lens elements comprising a first lens element 120, a second lens element 122, and a third lens element 123. The first lens element 120 and the third lens element 123 are partially overlapping, and the second lens element 122 and the third lens element 123 are partially overlapping. Thus, the incoming collimated beam is divided up into a first probing beam 126, a second probing beam 127, and a third probing beam 128 as shown in Fig 8.

The probing beams are reflected by the cantilever 130 and collected by the diffractive optical element, which re-collimates the probing beams and directs the beams towards the detector array 132 comprising a first overlapping area 154 and thus a first interference signal, as well as a second overlapping area 155 and thus a second interference signal. The first interference signal is measured by at least a first detector 134, and the second interference signal is measured by a first additional detector 135. Similar to the first embodiment, quadrature signals are measured by a second detector 136 and a second additional detector 137.

By properly choosing the distance between the probing beams, it is possible to obtain two separate interference measurements (between light reflected from adjacent beams), where the two interference measurements have different spatial frequency dependencies. By measuring the phase difference α between the two interference measurements, it is possible to expand the measurement range, in which an absolute differential displacement measurement can be obtained. This is shown in Fig. 19, wherein the two separate interference signals s₁₁ and s₁₂ (for instance measured by the first detector 134 and the second detector 135) are depicted as a function of the total differential deflection of the cantilever 130.

A similar principle is known from heterodyne interferometers utilising interference signals from light source with different wavelengths. Thus, the presently described interferometer may be perceived as a spatial heterodyne interferometer.

In an advantageous embodiment, a condensing lens is arranged between the beam splitter 116 and the detector array 132 so that the reflected beams are focused on to the detectors of the detector array 132. Furthermore, the cantilever array and/or the optical system can be arranged on a translational stage and/or a rotational stage so as to ensure that the probing beams are all focused onto the cantilevers of the cantilever array.

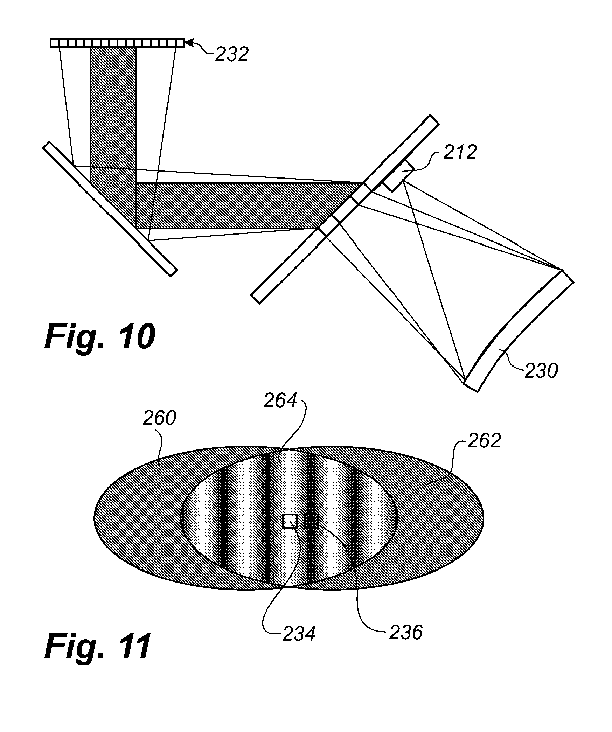

Fig. 10 shows a third embodiment of a detector system according to the invention, wherein like numerals refer to like parts of the first embodiment shown in Fig. 1 and 2. Therefore, only the difference between the two embodiments is described. In this embodiment, the light source means comprises a single light source 212, such as a laser diode, only. The light source is arranged so as to illuminate substantially the entire cantilever array comprising the cantilevers 230. Each cantilever 230 is imaged onto the detector array 232 as a first image 260 and a second image 262 of the cantilever as shown in Fig 11, the two images 260, 262 being partially overlapping so that an interference pattern 264 is formed at the overlapping region of the detector array 232. By properly choosing the spatial positions of the first detector 234 and the second detector 236, it is ensured that two quadrature signals are measured and thus a high sensitivity of the system.

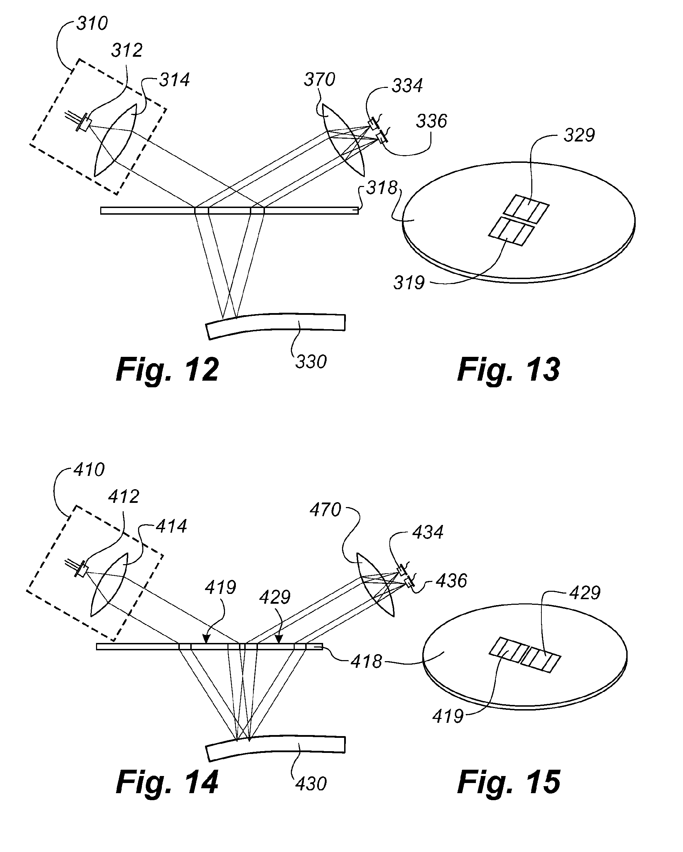

Fig. 12 and Fig. 13 show a fourth embodiment of a detector system according to the invention, wherein like numerals refer to like parts of the first embodiment shown in Figs. 1 and 2. Therefore, only the difference between the two embodiments is described. In this embodiment, the diffractive optical element layer 318 comprises a separate transmitter diffractive optical element 319 and a separate receiver diffractive optical element 329. The diffractive optical elements are adapted so as to separate the transmitter and receiver paths. Thereby, the beam splitter of the previous embodiment can be avoided. Consequently, the losses of the system can be minimised. The diffractive optical elements 319, 329 are in this embodiment adapted to focus (and re-collimate) the probing beams in a slight skew angle so that all of the reflected light from the probing beams is collected by the receiver diffractive optical element 329. The collected and re-collimated light is directed to a focusing lens 370, which focuses the overlapping reflected probing beams onto a first detector 334 and a second detector 336 detecting quadrature signals.

Fig. 14 and Fig. 15 show a fifth embodiment of a detector system according to the invention, wherein like numerals refer to like parts of the first embodiment shown in Figs. 1 and 2. Therefore, only the difference between the two embodiments is described. Similar to the fourth embodiment, the diffractive optical element layer 418 comprises a separate transmitter diffractive optical element 419 and a separate receiver diffractive optical element 429. In this embodiment, the diffractive optical elements are adapted to split up and focus (and re-collimate) an incoming collimated beam into a number of probing beams directed from the diffractive optical element layer 418 to the cantilevers 430 in a slight off-angle to the normal of layer 418. Substantially all the reflected light is received by the receiver diffractive optical element 429. The collected and re-collimated light is directed to a focusing lens 470, which focuses the overlapping reflected probing beams onto a first detector 434 and a second detector 436 detecting quadrature signals.

The invention has been described with reference to a preferred embodiment. However, the scope of the invention is not limited to the illustrated embodiment, and alterations and modifications can be carried out without deviating from the scope of the invention. For instance, the cantilever plane and the diffractive optical element layer are shown in the embodiments as being substantially parallel, and the incoming collimated beam is diverted approximately 45 degrees to the probing beams. However, it is also possible to transmit the probing beams at an angle to the normal of the diffractive optical element layer, e.g. 45 degrees. This has the advantage that the efficiency of the system can be increased even further due to the large diffraction angle of 90 degrees or more. However, since the diffractive optical element layer and the cantilever plane are not parallel in such embodiments, the overlapping lens elements of the diffractive optical element should be provided with different focal lengths in order to accommodate for the different distances to the cantilevers.

### List of reference numerals

- 10, 10', 110, 210, 310, 410: light source means
- 12, 12', 112, 212, 312, 412: light source / VCSEL array / laser diode array
- 14, 14', 114, 314, 414: collimating lens
- 16, 116, 216,: beam splitter
- 18, 118, 218, 318, 418: diffractive optical element layer
- 119: diffractive optical element
- 319, 419: diffractive optical transmitter element
- 20, 120: first lens element
- 22, 122: second lens element
- 123: third lens element
- 24: overlapping region
- 26: first probe beam
- 127: third probe beam
- 28: second probe beam
- 319, 419: diffractive optical receiver element
- 30, 30', 30", 130, 230, 330, 430: cantilever
- 31: cantilever axis
- 32, 32', 132, 232: detector array
- 34, 34', 134, 234, 334, 434: first detector
- 135: first additional detector
- 36, 136, 236, 336, 436: second detector
- 137: second additional detector
- 38', 38": first area of cantilever / first probe beam spot
- 39', 39": second area of cantilever / second probe beam spot
- 50: first reflected probe beam
- 52: second reflected probe beam
- 54, 154: overlapping beams
- 155: additional overlapping beams
- 56, 156: sidebands
- 60', 260: first image of probing beam spots / cantilever
- 62', 262: second image of probing beam spots / cantilever
- 64': detector measuring no signal
- 68': detector measuring no interference signal
- 370, 470: focusing lens
- 80: sample chamber
- 81: sample chamber outlet
- 82: buffer chamber
- 83: buffer chamber outlet
- 84: valve
- 85: detection chamber
- 86: inlet
- 87: outlet (to sample collection chamber)
- 88, 89: piston
- 90: collimating lens
- 92: grating
- 94: negative signal response
- 96: positive signal response
- 97: first time zone
- 98: second time zone

## Claims

1. A detector system for identifying substances in a sample, wherein the detector system comprises:
- a plurality of cantilevers (30) arranged in a cantilever array, the cantilevers (30) being arranged so as to be able to bend along a cantilever axis, wherein at least a number of the cantilevers (30) are provided with a recognition layer for identifying a substance in the sample, each of the number of cantilevers (30) being adapted to bend along its cantilever axis if the recognition layer reacts to a substance corresponding to the recognition layer, and
- an optical system for probing a deflection of the cantilevers, **characterised in that** the optical system includes a common-path interferometer setup comprising a transmitter part and a receiver part,
- the transmitter part comprising:
- light source means (10) for emitting spatial coherent light and arranged so as to illuminate at least parts of the cantilevers (30) of the cantilever array, and
- the receiver part comprising:
- a detector array (32), and
- a receiver diffractive optical element (19, 119, 329, 429) adapted so as to collect light reflected from the cantilever array and for each cantilever (30) forming at least two spatially overlapping images (60', 260; 62', 262) of said parts of the cantilever (30) so that interference between the two spatially overlapping images (60', 260; 62', 262) is formed at and measured by at least one separate detector (34) of the detector array (32).

2. A detector system according to claim 1, wherein the light source means (210) are arranged so as to illuminate at least a substantial part of the cantilevers (230), and wherein the receiver diffractive element is adapted so as to, for each cantilever, form displaced images of said substantial part of the cantilever at the detector array (232).

3. A detector system according to claim 1, wherein the transmitter part further comprises:
- a transmitter diffractive optical element (19, 119, 319, 419) arranged so as to split the light beam up into a plurality of probing beam sets and direct them to the cantilevers of the cantilever array,
- each of the individual probing beam sets comprising a number of substantially parallel probing beams including at least a first probing beam (26) focused onto a first area of a corresponding cantilever (30), and a second probing beam (28) focused onto a second area of the corresponding cantilever (30), the first area and the second area being positioned at a distance from each other along the cantilever axis (31), and wherein
- the receiver diffractive optical element part comprises:
- a detector array, and
- a receiver diffractive optical element (19, 119, 329, 429) adapted so as to collect light from the probing beam sets reflected from the cantilever array and direct the collected light towards the detector array (32) so that, for each probing beam set, interference between the first reflected probing beam (50) and the second reflected probing beam (52) is measured by at least one separate detector.

4. A detector system according to any of the preceding claims, wherein at least one of the cantilevers is a reference cantilever.

5. A detector system according to any of the preceding claims, wherein the light source means comprises a laser array (12), preferably a VCSEL array or a laser diode array.

6. A detector system according to claim 6, wherein the light source means further comprises a collimating lens (14) arranged so as to provide a number of substantially collimated light beams.

7. A detector system according to any of the preceding claims, wherein the transmitter diffractive optical element or elements are arranged in a first plane, and the receiver diffractive optical element or elements are arranged in a second plane, e.g. arranged in a common plane.

8. A detector system according to claim 7, wherein the first plane and/or the second plane are arranged substantially parallel to a cantilever plane, in which the cantilevers are positioned.

9. A detector system according to any of the preceding claims, wherein the transmitter diffractive optical element and/or the receiver diffractive optical element comprises a first lens element (20) for forming the first probe beam and a second lens element (22) for forming the second probe beam, the first lens element (20) and the second lens element (22) being partially overlapping (24).

10. A detector system according to any of the preceding claims, wherein a single diffractive optical element is utilised as both the transmitter diffractive optical element and the receiver diffractive optical element.

11. A detector system according to any of the preceding claims, wherein the transmitter part comprises a transmitter path for incoming light and the receiver part comprises a receiver path for light reflected from the cantilevers, the transmitter path and the receiver path having an overlapping part, wherein the transmitter path and the receiver path are substantially overlapping.

12. A detector system according to any of the preceding claims, wherein the detector array is a CMOS array or a CCD array.

13. A detector system according to any of the preceding claims, wherein a number of the probing beam sets comprises a third probing beam (27) focused onto a third area of the corresponding cantilever, the third area being positioned at a first distance from the first area along the cantilever axis and at a second distance from the second area along the cantilever axis.

14. A detector system according to any of the preceding claims, wherein the cantilevers and/or the optical system are arranged on a rotational stage so that a cantilever plane comprising the cantilevers can be rotationally adjusted in relation to the transmitter diffractive optical element and the receiver diffractive optical element.

15. A detector system according to any of the preceding claims, wherein the cantilevers and/or the optical system are arranged on a transitional stage so that a distance between a cantilever plane comprising the cantilevers and the diffractive optical elements can be adjusted.
